# EUROPEAN PATENT SPECIFICATION

(11) **EP 0 837 634 B1**
(45) Date of publication and mention of the grant of the patent: **05.02.2003**
(21) Application number: 96923477.2
(22) Date of filing: 27.06.1996
(51) Int. Cl.: A01N 63/00, A01N 63/02, C12P 1/04, C12N 1/20

(54) **Novel Bacillus thuringiensis pesticidal substance**
Neue Bacillus thuringiensis Pestizidsubstanz
Nouvelle substance pesticide obtenue à partir du Bacillus thuringiensis

(30) Priority: 27.06.1995 US 543 P
(43) Date of publication of application: 29.04.1998
(73) Proprietor: Abbott Laboratories, Abbott Park, Illinois 60064-3500 (US)
(72) Inventor: MANKER, Denise, C., Davis, CA 95616 (US); LIU, Chi-Li, DECATUR, ILLINOIS 62526 (US); MACMULLAN, Anita, M., Davidson, NC 28036 (US)
(74) Representative: Modiano, Guido, Dr.-Ing.
(86) International application number: US9610959
(87) International publication number: WO97001282

(56) References cited:
- WO-A-94/09630
- WO-A-95/25181
- US-A- 5 204 100
- US-A- 5 369 027

## Description

### BACKGROUND OF THE INVENTION

### Field of the Invention

The invention relates to a novel substance having pesticidal activity, particularly against insect pests of the order Coleoptera. The invention further relates to pesticidal compositions comprising the substance as well as methods of using the pesticidal compositions to control a pest.

### Description of the Related Art

*Bacillus thuringiensis* is a motile, rod-shaped, gram-positive bacterium that is widely distributed in nature. During sporulation, *Bacillus thuringiensis* produces a parasporal crystal inclusion(s) which is insecticidal upon ingestion to susceptible insect larvae of the order Lepidoptera, Diptera, or Coleoptera. The inclusions may vary in shape, number, and composition. They are comprised of one or more proteins called delta-endotoxins, which may range in size from 27-140 kDa. The insecticidal delta-endotoxins are generally converted by proteases in the larval gut into smaller (truncated) toxic polypeptides, causing midgut destruction, and ultimately, death of the insect.

*Bacillus thuringiensis* crystal delta-endotoxins are the most widely used biopesticide. There are several *Bacillus thuringiensis* strains that are used as producers of crystal delta-endotoxins for the forestry, agricultural, and public health areas. *Bacillus thuringiensis* subsp. *kurstaki* and *Bacillus thuringiensis* subsp. *aizawai* produce delta-endotoxins specific for Lepidoptera. A delta-endotoxin specific for Coleoptera is produced by *Bacillus thuringiensis* subsp. *tenebrionis*. Furthermore, *Bacillus thuringiensis* subsp. *israelensis* produces delta-endotoxins specific for Diptera. Other *Bacillus thuringiensis* strains have been reported to produce delta-endotoxins pesticidal to nematodes, Acari, Hymenoptera, Phthiraptera, Platyhelminthes, Homoptera, Blattodea, and Protozoa.

*Bacillus thuringiensis* is also known to produce other water-soluble molecules which are pesticidal. For example, a heat-stable pesticidal adenine-nucleotide analog, known as beta-exotoxin or thuringiensin, is produced by a few *Bacillus thuringiensis* strains (Sebesta *et al*., in H.D. Burges (ed.), *Microbial Control of Pests and Plant Diseases,* Academic Press, New York p. 249-281, 1981). Beta-exotoxin has a molecular weight of 789 and is comprised of adenosine, glucose, and allaric acid (Lüthy *et al*., in Kurstak (ed.), *Microbial and Viral Pesticides,* Marcel Dekker, New York, 1982, pp. 35-72). Its host range includes, but is not limited to, *Musca domestica, Mamestra configurata* Walker, *Tetranychus urticae*, *Drosophila* *melanogaster*, and *Tetranychus cinnabarinus*. Beta-exotoxin may be classified as type I or type II beta-exotoxin (Levinson *et al*., 1990, *Journal of Bacteriology* 172:3172-3179). Beta-exotoxin type I was found to be produced by *Bacillus thuringiensis* subsp. *thuringiensis* serotype 1, *Bacillus thuringiensis* subsp. *tolworthi* serotype 9, and *Bacillus thuringiensis* subsp. *darmstadiensis* serotype 10. Beta-exotoxin type II was found to be produced by *Bacillus thuringiensis* subsp. *morrisoni* serotype 8ab and is active against *Leptinotarsa decemlineata*. Other water soluble substances that have been isolated *from Bacillus thuringiensis* include alpha-exotoxin with toxicity against the larvae of *Musca domestica* (Lüthy, 1980, *FEMS Microbiological Letters* 8:1-7); gamma-exotoxins, which are various enzymes including lecithinases, chitinases, and proteases, the toxic effects of which are expressed only in combination with beta-exotoxin or delta-endotoxin (Forsberg *et al*., 1976, *Bacillus thuringiensis*: *Its Effects on Environmental Quality*, National Research Council of Canada, NRC Associate Committee on Scientific Criteria for Environmental Quality, Subcommittees on Pesticides and Related Compounds and Biological Phenomena); sigma exotoxin has a structure similar to beta-exotoxin, and is also active against *Leptinotarsa decemlineata* (Argauer *et al*., 1991, *Journal of Entomological Science* 26:206-213); and anhydrothuringiensin (*Coll. Czechosslovak Chem. Comm*. 40, 1775, 1975).

Stonard *et al*. (1994, in *Natural and Engineered Pest Management Agents,* Paul A. mann, Robert M. Hollingworth, eds., American Chemical Society, Washington, D.C., pp. 25-36) discloses Diabroticins having the structure 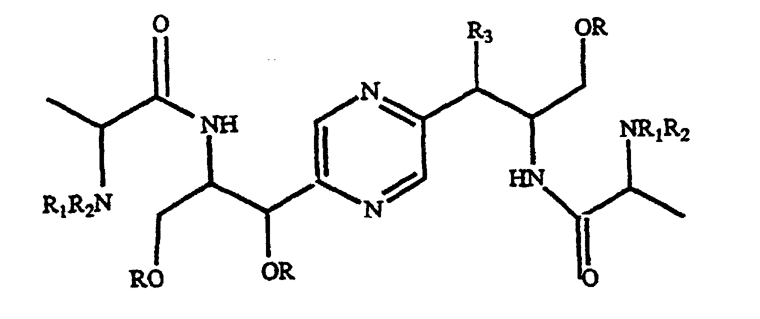
I R. R₁, R₂=H, R₃=OH Diabroticin A
2 R, R₁, R₂, R₃=H Diabroticin B
Diabroticins were isolated from *Bacillus subtilis* and have activity against *Diabrotica undecimpunctata, Leptinotarsa decemlineata, Anthomus grandis* Boheman, mosquito larva, *Staphylococcus aureus,* and *Micrococcus lutea*, but did not have activity against European corn borer, *Escherichia coli, Bacillus subtilis*, and *Pseudomonas aeruginosa.* Activity against *other* pests was not disclosed in Stonard *et al*. Diabroticin A was also isolated from fermentation broths of *Bacillus cereus*.

The art has strived to identify new biopesticides for replacing chemical pesticides which are detrimental to the environment or pesticidally ineffective as a result of a particular pest developing resistance to the pesticide.

It is an object of the present invention to provide a novel substance with pesticidal activity against insect pests of the order of Coleoptera.

### SUMMARY OF THE INVENTION

The present invention relates to a novel substance obtained from a supernatant of a culture broth of a *Bacillus* strain wherein the substance has activity against an insect pest of the order Coleoptera; 1H NMR shifts of about δ 5.17 (d,1H), 4.48 (d, 1H), 4.1 (m, 1H), 4.0 (dd, 1H), 3.92 (d, 1H), 3.85 (m, 2H), 3.80 (m, 1H), 3.65 (m, 1H), 3.50 (m, 1H), 3.36 (m, 1H), 3.1 (dd, 1H), 2.8 (m, 1H), 1.27 (d, 3H), and 1.19 (m, 3-4H); 12 carbons; and UV end absorption. The substance of the present invention may also act, for example, as a potentiator or synergizer together with a different *Bacillus* related pesticide against a pest.

The present invention also relates to a mutant of the *Bacillus* strain wherein the amount of the substance produced by the mutant is greater than the amount of the substance produced by a corresponding parental strain. The present invention is further directed to methods for obtaining such a mutant comprising (a) treating the *Bacillus* strain with a mutagen; (b) growing the mutated *Bacillus* strain of step (a) in a medium suitable for selecting the mutant strain; and (c) selecting the mutant strain of step (b) on the basis of increased production of the substance.

The present invention also relates to a method for obtaining a "substantially pure" substance of the present invention comprising (a) culturing *a Bacillus* strain under suitable conditions; (b) recovering a supernatant of the culture of the *Bacillus* strain of step (b); and (c) isolating the substance from the supernatant of step (b) to obtain the substantially pure substance.

The present invention further relates to pesticidal compositions comprising the substance and a pesticidal carrier as well as the substance and a *Bacillus* related pesticide, a chemical pesticide and/or an entomopathogenic virus, as well as methods of using the pesticidal compositions to control a pest.

### BRIEF DESCRIPTION OF THE FIGURES

Figure 1 is the ¹H NMR spectrum of the substance of the present invention.
Figure 2 is the 13C NMR spectrum of the substance of the present invention.

### DETAILED DESCRIPTION OF THE INVENTION

The present invention is directed to a substance having pesticidal activity, particularly against insect pests of the order Coleoptera. "Pesticidal activity" is defined herein as a measure of the amount of activity of the substance of the present invention against a pest through killing or stunting of the growth of the pest or protecting a plant from pest infestation. In a specific embodiment, the substance of the present invention has a molecular weight in the range of about 250 to about 500; ¹H NMR shifts of about δ 5.17 (d,1H), 4.48 (d, 1H), 4.1 (m, 1H), 4.0 (dd, 1H), 3.92 (d, 1H), 3.85 (m, 2H), 3.80 (m, 1H), 3.65 (m, 1H), 3.50 (m, 1H), 3.36 (m, 1H), 3.1 (dd, 1H), 2.8 (m, 1H), 1.27 (d, 3H), and 1.19 (m, 3-4H); 12 carbons with ¹³C NMR shifts of about δ 97.8, 92.5, 71.1, 68.1, 67.8, 63.6, 57.8, 54.7, 52.9, 52.7, 52.3, and 19.7; and UV end absorption. In another specific embodiment, the substance of the present invention is water-soluble.

The pesticidal activity of the substance against an insect pest may be assayed using procedures known in the art, such as artificial diet incorporation, artificial diet overlay, leaf painting, leaf dip, and foliar spray.

In a specific embodiment, the substance of the present invention has pesticidal activity against an insect pest of the order Coleoptera. In a more specific embodiment, the substance of the present invention has pesticidal activity against an insect pest of the genus *Diabrotica* of the order Coleoptera. In a most specific embodiment, the substance of the present invention has pesticidal activity against *Diabrotica undecimpunctata* of the order Coleoptera.

The substance of the present invention may be recovered from the supernatant of a *Bacillus* fermentation. In a specific embodiment, the substance of the present invention is obtained from the fermentation supernatant of a *Bacillus thuringiensis* strain. In a more specific embodiment, the substance of the present invention is obtained from the fermentation supernatant of a *Bacillus thuringiensis* strain EMCC-0080 having the identifying characteristics of NRRL B-21093, or mutants thereof having substantially the same properties of EMCC-0080, disclosed in WO 95/25181 (published 21.9.1995) and WO 96/28391 (published 19.9.1996).

The present invention is also directed to a mutant of a *Bacillus* strain wherein the amount of the substance produced by the mutant is greater than the amount of the substance produced by a corresponding parental strain. The present invention is further directed to methods for obtaining such a mutant. A "parental strain" as defined herein is the original *Bacillus* strain before mutagenesis which leads to the mutated strain.

In one embodiment, the substance of the present invention is obtained from a mutant of a *Bacillus* strain, particularly, a *Bacillus thuringiensis* strain, wherein the substance is produced in an amount greater than the amount of the substance produced by a corresponding parental strain. To obtain such mutants, the parental strain may, for example, be treated with a mutagen by chemical means such as N-methyl-N'-nitro-N-nitrosoguanidine or ethylmethanesulfonate, or by irradiation with gamma-ray, X-ray, or UV. Specifically, one method of mutating *a Bacillus* strain and selecting such a mutant comprises the following procedure:
i) the parental strain is treated with a mutagen;
ii) the thus presumptive mutants are grown in a medium suitable for selection of a mutant strain; and
iii) the mutant strain is selected on the basis of increased production of the substance of the present invention.

According to a preferred embodiment of this method, the selected colonies are grown in a normal production medium, and a final selection for such mutants is performed.

*Bacillus thuringiensis* may be cultured using media and fermentation techniques known in the art (see, for example, Rogoff *et al*., 1969, *Journal of Invertebrate Pathology* 14:122-129; Dulmage *et al*., 1971, *Journal of Invertebrate Pathology* 18:353-358; Dulmage *et al*., in *Microbial Control of Pests and Plant Diseases*, H.D. Burges, ed., Academic Press, N.Y., 1980). Upon completion of the fermentation cycle, the supernatant can be recovered by separating *Bacillus thuringiensis* spores and crystals from the fermentation broth by means well known in the art, e.g., centrifugation and/or ultrafiltration. The substance of the present invention is contained in the supernatant which may be recovered by means well known in the art, e.g., ultrafiltration, evaporation, and spray-drying.

The present invention also relates to a method for obtaining a "substantially pure" substance of the present invention. A "substantially pure" substance is defined herein as a substance which contains less than 5% contaminants, for example, delta-endotoxin protein. Purification of the substance of the present invention can be carried out by various procedures known in the art including, but not limited to, chromatography (e.g., ion exchange, affinity, and size exclusion column chromatography), electrophoretic procedures, differential solubility, extraction, or any other standard technique known in the art (see, for example, *Protein Purification, eds.* J-C. Janson and Lars Ryden, VCH Publishers, New York, 1989). Specifically, one method for obtaining a substantially pure substance of the present *invention* comprises the following procedure:
(i) culturing *a Bacillus* strain under suitable conditions;
(ii) recovering a supernatant of the culture of the *Bacillus* strain; and
(iii) isolating the substance from the supernatant to obtain the substantially pure substance.

The present invention is further directed to pesticidal compositions comprising the substance in an effective amount to control a pest and a pesticidal carrier. "Effective amount" is defined herein as the amount of the substance sufficient to control a pest through killing or stunting of the growth of the pest or protecting a plant from pest infestation. The pesticidal compositions may comprise the substance of the present invention in a substantially pure form or as a supernatant from a whole broth culture of a *Bacillus* strain in dry, concentrated, or liquid form and a suitable pesticidal carrier, examples of which are disclosed *infra*. The substance is present in the composition at a concentration of from about 0.001% to about 60% (w/w).

The pesticidal compositions of the present invention may further comprise a deposition agent which assists in preventing the composition from drifting from the target area during application (e.g., as it is sprayed from a plane), or from being blown away from the plant once it has been deposited. The deposition agent in the compositions of the present invention is preferably a proteinaceous material, which has the added benefit of being palatable to the insect. Any animal or vegetable protein is suitable for this purpose, in dry or in liquid form. Examples of useful sources of protein which can be conveniently and economically added to the composition include, but are not limited to, soy protein, potato protein, soy flour, potato flour, fish meal, bone meal, yeast extract, and blood meal. Alternative deposition agents include modified cellulose (carboxymethylcellulose), botanicals (grain flours, ground plant parts), non-phyllosilites (talc, vermiculite, diatomaceous earth), natural clays (attapulgite, bentonite, kaolinite, montmorillonite), and synthetic clays (Laponite). When utilized, the deposition agent is present in the pesticidal compositions of the present invention in an amount of between about 0.4% w/w and about 50% w/w, preferably between about 1% w/w and about 20% w/w.

The pesticidal compositions of the present invention may further comprise an antifreeze/humectant agent which suppresses the freeze point of the product and helps minimize evaporation when sprayed and which maintains deposit texture making the product more efficacious and palatable. Examples of antifreeze/humectant agents include, but are not limited to, ethylene glycol, propylene glycol, dipropylene glycol, glycerol, butylene glycols, pentylene glycols and hexylene glycols. When utilized, the antifreeze/humectant agent is present in the pesticidal compositions of the present invention in an amount of between about 0.5% w/w and about 25% w/w, preferably between about 2% w/w and about 15% w/w.

The pesticidal compositions of the present invention may further comprise a surfactant in an amount where it acts as an emulsifying, a wetting, or a dispersing agent. Examples of such surfactants are anionic surfactants such as carboxylates, for example, a metal carboxylate of a long chain fatty acid; N-acylsarcosinates; mono or di-esters of phosphoric acid with fatty alcohol ethoxylates or salts of such esters; fatty alcohol sulphates such as sodium dodecyl sulphate, sodium octadecyl sulphate or sodium cetyl sulphate; ethoxylated fatty alcohol sulphates; ethoxylated alkylphenol sulphates; lignin sulphonates; petroleum sulphonates; alkyl aryl sulphonates such as alkyl-benzene sulphonates or lower alkylnaphthalene sulphonates, e.g., butyl naphthalene sulphonate; salts or sulphonated naphthalene-formaldehyde condensates; salts of sulphonated phenol-formaldehyde condensates; or more complex sulphonates such as amide sulphonates, e.g., the sulphonated condensation product of oleic acid and N-methyl taurine or the dialkyl sulphosuccinates, e.g., the sodium sulphonate or dioctyl succinate. Further examples of such surfactants are non-ionic surfactants such as condensation products of fatty acid esters, fatty alcohols, fatty acid amides or fatty-alkyl- or alkenyl-subsdtuted phenols with ethylene oxide, block copolymers of ethylene oxide and propylene oxide, acetylenic glycols such as 2,4,7,9-tetraethyl-5-decyn-4,7-diol, or ethoxylated acerylenic glycols. Further examples of such surfactants are cationic surfactants such as aliphatic mono-, di-, or polyamine as acetates, naphthenates or oleates; oxygen-containing amines such as an amine oxide of polyoxyethylene alkylamine; amide-linked amines prepared by the condensation of a carboxylic acid with a di- or polyamine; or quaternary ammonium salts. When utilized, the surfactant is present in an amount of between about 0.5% w/w and about 25% w/w, preferably between about 1% w/w and about 8% w/w.

The pesticidal compositions of the present invention may further comprise an inert material. Examples of inert materials include inorganic minerals such as diatomaceous earth, kaolin, mica, gypsum, fertilizer, phyllosilicates, carbonates, sulfates, or phosphates; organic materials such as sugars, starches, or cyclodextrins; or botanical materials such as wood products, cork, powdered corncobs, rice hulls, peanut hulls, and walnut shells.

The pesticidal compositions according to the present invention may further comprise a preservative, a feeding stimulant, an attractant, an encapsulating pesticide, a binder, a dye, an ultraviolet light protectant, a buffer, a flow agent, or other component to facilitate product handling and application for particular target pests.

The pesticidal compositions of the present invention may further comprise a *Bacillus* related pesticide, particularly, a *Bacillus thuringiensis* biopesticide. *The Bacillus thuringiensis* biopesticide may be derived from, but not limited to, *Bacillus thuringiensis* subsp. *aizawai, Bacillus thuringiensis* subsp. *alesti, Bacillus thuringiensis* subsp. *canadiensis, Bacillus thuringiensis* subsp. *colmeri, Bacillus thuringiensis* subsp. *coreanensis, Bacillus* *thuringiensis* subsp. *dakota, Bacillus thuringiensis* subsp. *darmstadiensis, Bacillus thuringiensis* subsp. *dendrolimus, Bacillus thuringiensis* subsp. *entomocidus, Bacillus thuringiensis* subsp. *finitimus, Bacillus thuringiensis* subsp. *galleriae, Bacillus thuringiensis* subsp. *indiana, Bacillus thuringiensis* subsp. *israelensis, Bacillus thuringiensis* subsp. *kenyae, Bacillus thuringiensis* subsp. *kumamotoensis, Bacillus thuringiensis* subsp. *kurstaki, Bacillus thuringiensis* subsp. *kyushuensis, Bacillus thuringiensis* subsp. *japonensis, Bacillus thuringiensis* subsp. *mexcanensis, Bacillus thuringiensis* subsp. *morrisoni, Bacillus thuringiensis* subsp. *neoleonensis, Bacillus thuringiensis* subsp. *nigeriae, Bacillus thuringiensis* subsp. *ostriniae, Bacillus thuringiensis* subsp. *Pakistani, Bacillus thuringiensis* subsp. *pondicheriensis, Bacillus thuringiensis* subsp. *shandongiensis, Bacillus thuringiensis* subsp. *silo, Bacillus thuringiensis* subsp. *sotto, Bacillus thuringiensis* subsp, *subtoxicus, Bacillus thuringiensis* subsp. *tenebrionis, Bacillus thuringiensis* subsp. *thompsoni, Bacillus thuringiensis* subsp. *tochigiensis, Bacillus thuringiensis* subsp. *tohokuensis, Bacillus thuringiensis* subsp. *tolworthi*, *Bacillus thuringiensis* subsp. *toumanoffi*, *Bacillus thuringiensis* subsp, *wuhanensis*, or *Bacillus thuringiensis* subsp. *yunnanensis.* Depending on the recommended use rate for *a Bacillus thuringiensis* biopesticide formulated product, the substance of the present invention may be combined either with the *Bacillus thuringiensis* product in a tank mix or in the formulated product

The *Bacillus thuringiensis* biopesticide, i.e., a delta-endotoxin protein or a pesticidally-active fragment thereof, may be selected from the group including, but not limited to, CryI, CryII, CryIII, CryIV, CryV, and CryVI. More specifically, the *Bacillus thuringiensis* delta-endotoxin protein or pesticidally-active fragment thereof may include, but is not limited to, CryIA(a), CryIA(b), CryIA(c), CryIB, CryIC, CryID, CryIE, CryIF, CryIIA, CryIIB, CryIIIA, CryIIIB, CryIIIC, CryIVA, CryIVB, CryIVC, CryIVD, CryV, CryVI, and CytA. It is also within the scope of the present invention that the *Bacillus thuringiensis* biopesticide may also comprise a spore derived from the *Bacillus thuringiensis* strain.

The *Bacillus thuringiensis* biopesticide in the pesticidal compositions of the present invendon alternatively may be derived from a cell wherein a gene, which encodes a *Bacillus thuringiensis* delta-endotoxin protein or pesticidally-active fragment thereof, has been inserted. Furthermore, within the scope of the present invention, the *Bacillus thuringiensis* biopesticide or pesticidally-active fragment thereof may be derived from a transconjugate strain wherein a plasmid containing a gene, which encodes the *Bacillus thuringiensis* delta-endotoxin protein or pesticidally-active fragment thereof, has been transferred by cell-cell conjugation.

The pesticidal compositions of the present invention may comprise the substance of the present invention in an amount between about 0.001 and about 100 grams per gram of *Bacillus thuringiensis* biopesdcide.

The pesticidal compositions of the present invention may further comprise one or more synergists, potentiators, or phagostimulants known in the art. For example, the potentiator disclosed in WO 94/09630 or a pesticidally-active salt thereof may be used in the compositions of the present invention.

The compositions of the present invention may further comprise an entomopathogenic virus. Examples of such entomopathogenic viruses include, but are not limited to, *Autographa californica* nuclear polyhedrosis virus (NPV), *Syngrapha falcifera* NPV, *Cydia pomonella* granulosis virus (GV), *Heliothis zea* NPV, *Lymantria dispar* NPV, *Orgyia pseudotsugata* NPV, *Spodoptera exigua* NPV, *Neodiprion lecontei* NPV, *Neodiprion sertifer* NPV, *Harrisina brillians* NPV, and *Endopiza viteana* Clemens NPV.

The compositions of the present invention may further comprise a chemical pesticide. Examples of such chemical pesticides include, but are not limited to, insect growth regulators, carbamates, organophosphates, pyrethroids, inorganic fluorines, pyrazoles, pyrroles, and avermectins.

The present invention is further directed to a method for controlling pest infestation on plants comprising applying to a plant pesticidal compositions of the present invention.

The pesticidal compositions of the present invention can be applied in a dry or liquid form, e.g., a suspension. a solution, an emulsion, a dusting powder, a dispersible granule, a wettable powder, an emulsifiable concentrate, an aerosol or impregnated granule, or a concentrate or primary composition which requires dilution with a suitable quantity of water or other diluent before application. The concentrations of each component in the composition will vary depending upon the nature of the particular composition, specifically, whether it is a concentrate or to be used directly. The composition may contain about 1% to about 98% of a solid or liquid inert carrier. The compositions will be preferably administered at the labeled rate for commercial producs, preferably about 0.01 pound to 5.0 pounds per acre when in dry form and at about 0.01 pint to 25 pints per acre when in liquid form.

The pesticidal compositions of the present invention can be applied directly to a plant by, for example, spraying or dusting at the time when the pest has begun to appear on the plant or before the appearance of pests as a protective measure. The pesticidal compositions can be applied by foliar, furrow, broadcast granule, "lay-by", or soil drench application. The compositions of the present invention can also be applied directly to ponds, lakes, streams, rivers, still water, and other areas subject to infestation by pests of concern to public health. The compositions can be applied by spraying, dusting, sprinkling, or the like. The spray or dust can conveniently contain another pesticide. The pesticidal compositions of the present invention are preferably applied directly to the plant.

The pesticidal compositions of the present invention can be applied to protect a number of different plant types, including, but not limited to, cereals (wheat, barley, rye, oats, rice, sorghum and related crops), beets (sugar beet and fodder beet), drupes, pomes and soft fruit (apples, pears, plums, peaches, almonds, cherries, strawberries, raspberries, and blackberries), leguminous plants (alfalfa, beans, lentils, peas, soybeans), oil plants (rape, mustard, poppy, olives, sunflowers, coconuts, castor oil plants, cocoa beans, groundnuts), cucumber plants (cucumber, marrows, melons), fibre plants (cotton, flax, hemp, jute), citrus fruit (oranges, lemons, grapefruit, mandarins), vegetables (spinach, lettuce, asparagus, cabbages and other brassicae, carrots, onions, tomatoes, potatoes), lauraceae (avocados, cinnamon, camphor), deciduous trees and conifers (linden-trees, yew-trees, oak-trees, alders, poplars, birch-trees, firs, larches, pines), or plants such as maize, turf plants, tobacco, nuts, coffee, sugar cane, tea, vines, hops, bananas and natural rubber plants, as well as ornamentals.

The present invention further relates to a method for applying the substance of the present invention to a transgenic plant, which contains a gene that encodes a *Bacillus thuringiensis* biopesticide.

The pesticidal compositions of the present invention can be used in the treatment or prevention of infestations of a number of different insect types. It is particularly preferred to use the compositions of the present invention to eliminate pests of the order Coleoptera, e.g., *Leptinotarsa sp., Acanthoscelides obtectus, Callosobruchus chinensis, Epilachna varivestis, Pyrrhalta luteola, Cylas formicarius elegantulus, Listronotus oregonensis, Sitophilus sp., Cyclocephala borealis, Cyclocephala immaculata, Macrodactylus subspinosus*, *Popillia japonica, Rhizotrogus majalis, Alphitobius diaperinus, Palorus ratzeburgi, Tenebrio molitor, Tenebrio obscurus, Tribolium castaneum, Tribolium confusum,* aud *Tribolius destructor*. However, the pesticidal compositions of the present invention may also be effective against insect pests of the order Lepidoptera, e.g., *Achroia grisella, Acleris gloverana, Acleris variana, Adoxophyes orana, Agrotis ipsilon, Alabama argillacea, Alsophila pometaria, Amyelois transitella, Anagasta kuehniella, Anarsia lineatella, Anisota senatoria, Antheraea pernyi, Anticarsia gemmatalis, Archips sp., Argyrotaenia sp., Athetis mindara, Bombyx mori, Bucculatrix thurberiella, Cadra cautella, Choristoneura sp., Cochylis hospes, Colias eurytheme, Corcyra cephalonica, Cydia latiferreanus, Cydia pomonella, Datana integerrima, Dendrolimus sibericus, Desmia funeralis, Diaphania hyalinata, Diaphania nitidalis, Diatraea grandiosella, Diatarea saccharalis, Ennomos subsignaria, Eoreuma loftini, Ephestia elutella, Erannis tiliaria, Estigmene acrea, Eulia salubricola, Eupoecilia ambiguella, Euproctis chrysorrhoea, Euxoa messoria, Galleria mellonella, Grapholita molesta*, *Harrisina americana, Helicoverpa subflexa, Helicoverpa zea, Heliothis virescens, Hemileuca oliviae, Homoeosoma electellum, Hyphantria cunea, Keiferia lycopersicella, Lambdina fiscellaria fiscellaria, Lambdina fiscellaria lugubrosa, Leucoma salicis, Lobesia botrana, Loxostege sticticalis, Lymantria dispar, Macalla thyrsisalis, Malacosoma sp., Mamestra brassicae, Mamestra configurata, Manduca quinquemaculata, Manduca sexta, Maruca testulalis, Melanchra picta, Operophtera brumata, Orgyia sp., Ostrinia nubilalis, Paleacrita vernata, Papilio cresphontes, Pectinophora gossypiella, Phryganidia californica, Phyllonorycter blancardella, Pieris napi, Pieris rapae, Plathypena scabra, Platynota flouendana, Platynota sultana, Platyptilia carduidactyla, Plodia interpunctella, Plutella zylostella, Pontia protodice, Pseudaletia* *unipuncta, Pseudoplusia includens, Sabulodes aegrotata, Schizura concinna, Sitotroga cerealella, Spilonota ocellana, Spodoptera sp., Thaurnstopoea pityocampa, Tineola bisselliella, Trichoplusia ni, Udea rubigalis, Xylomyges curiails, and Yponomeuta padella*; *the order* Diptera, e.g., *Aedes sp., Andes vittatus*, *Anastrepha ludens, Anastrepha suspensa, Anopheles barberi, Anopheles quadrimaculatus, Armigeres subalbatus, Calliphora stygian, Calliphora vicina, Ceratitis capitata, Chironomus tentans, Chrysomya rufifacies, Cochliomyia macellaria, Culex sp., Culiseta inornata, Dacus oleae, Delia antiqua, Delia platura, Delia radicum, Drosophila melanogaster, Eupeodes corollae, Glossina austeni, Glossina brevipalpis, Glossina fuscipes, Glossina morsitans centralis, Glossina morsitans morsitans, Glossina moristans submorsitans, Glossina pallidipes, Glossina palpalis gambiensis, Glossina palpalispalpalis, Glossina tachinoides, Haemagogus equinus, Haematobia irritans, Hypoderma bovis, Hypoderma lineatum, Leucopis ninae, Lucilia cuprina, Lucilia sericata. Lutzomyia longlpaipis, Lutzomyia shannoni, Lycoriella mali, Mayetiola destructor, Musca autumnalis, Musca domestica, Neobellieria sp., Nephrotoma suturalis, Ophyra aenescens, Phaenicia sericata, Phlebotomus sp., Phormia regina, Sabethes cyaneus, Sarcophaga bullata, Scatophaga stercoraria, Stomoxys calcitrans, Toxorhynchites amboinensis,* and *Tripteroides bambusa*; the order Acari, e.g., *Oligonychus pratensis, Panonychus ulmi, Tetranychus urticae*; the order Hymenoptera, e.g., *Iridomyrmex humilis,* and *Solenopsis invicta*; the order Isoptera, e.g., *Reticulitermes hesperus, Reticulitermes flavipes, Coptotermes formosanus, Zaotermopsis angusticollis, Neotermes connexus, Incisitermes minor, and Incisitermes immigrans*; *the order* Siphonaptera, e.g., *Ceratophyllus gallinae, Ceratophyllus niger, Nosopsyllus fasciatus, Leptopsylla segnis, Ctenocephalides canis, Ctenocephalides felis, Echicnophaga gallinacea, Pulex irritans, Xenopsylla cheopis, Xenopsylla vexabilis,* and *Tunga penetrans*; and the order Tylenchida, e.g., *Melodidogyne incognita* and *Protylenchus penetrans.*

The present invention is further described by the following examples which are not construed as limiting the scope of the invention.

### EXAMPLES

### EXAMPLE 1: Cultivation of Bacillus thuringiensis EMCC-0080

*Bacillus thuringiensis* strain EMCC-0080 is grown for 24 hours at 30°C in a medium adjusted to pH 7.0 with the following composition:

| | |
|---|---|
| Hydrolyzed Starch | 40 g/l |
| Vegetable Protein | 40 g/l |
| KH₂PO₄ | 1.8 g/l |
| K₂HPO₄ | 4.5 g/l |
| MgSO₄-7H₂O | 0.3 g/l |
| Trace metals | 0.2 ml |

Cells and other insolubles are removed from the whole culture broth of *Bacillus thuringiensis* strain EMCC-0080 by centrifugation followed by filtration of the resulting supernatant through Celite and a 0.2 µ membrane. The resulting permeate is then concentrated 10-fold by evaporation.

### EXAMPLE 2: Diabrotica undecimpunctata Bioassay

Samples of 20 µl are applied to individual wells of a 96 well microtiter plate containing 200 µl of solidified artificial insect diet per well, and then air dried. One neonate of *Diabrotica undecimpunctata* is gently placed into each well with a small paint brush. The microtiter plates are then sealed with Mylar punched with holes for air exchange and are incubated at 30°C and 80% humidity. Scoring for percent mortality is carried out at 5-6 days

### EXAMPLE 3: Purification of the Pesticidal Substance

The purification of the pesticidal substance from the 10X concentrated permeate of Example 1 is achieved using a three step purification procedure. During purification, activity is monitored by the *Diabrotica undecimpunctata* surface bioassay as described in Example 2. All chromatographic steps employ detection at 278 nm unless otherwise noted.

In the first step, the 10X concentrated permeate is first purified by Pharmacia SP Sepharose Fast Flow (cation exchange) column chromatography (5 x 23 cm). The column is washed with 1350 ml of deionized water followed by 1350 ml of 20 mM ammonium acetate buffer at pH 5.0. The column is then washed with 100 ml of 1 M ammonium acetate buffer at pH 5.0 and re-equilibrated with 20 mM ammonium acetate buffer at pH 5.0 before loading the sample. A 425 ml sample of the 10X concentrated permeate is diluted to 18 liter with deionized water to obtain a conductivity of 2.01 mhos and a pH of 4.8. The diluted sample is loaded at a rate of 17 ml per minute onto the column pie-equilibrated with 20 mM ammonium acetate buffer at pH 5.0. After loading of the sample, the column is washed with 3 liters of 20 mM ammonium acetate buffer at pH 5.0. The column is eluted at 18 ml per minute with a 5.0 liter continuous gradient from 20 mM to 1.0 M ammonium acetate buffer at pH 5.0. Fractions are collected every minute and and every fifth fraction is bioassayed against *Diabrotica undecimpuncta as* described above. Activity is found by bioassay to reside in fractions 25-95. The active fractions are pooled, lyophilized, and redissolved in 50 ml of deionized water.

In the second step, the 50 ml pool from the first step is loaded onto a BioRad P2 (extra fine) size exclusion column (5 x 100 cm) which is pre-equilibrated with deionized water. The column is eluted at a flow rate of 1 ml per minute with deionized water. Fractions are collected every 10 minutes and bioassayed (every 5th faction) as before. Activity is found by bioassay to reside in fractions 94-120 which are pooled.

In the third step. a 6 ml sample of the pooled active fractions from step 2 is diluted to 10 ml with deionized water to obtain a conductivity of 223 mhos. The diluted sample is loaded onto a TSK SP-5PW HPLC column (15 cm x 21.5 mm ID) and then eluted at 4 ml per minute using a 120 ml continuous gradient from 225 mM to 400 mM ammonium acetate buffer at pH 5.0. Fractions are monitored at 310 nm and collected every 1.5 minutes and bioassayed (every fifth fraction) as before. At approximately 15 minutes, a large peak eluted followed by a shoulder peak which contained the active substance as determined by bioassay.

### EXAMPLE 4: Characterization of Pesticidal Substance

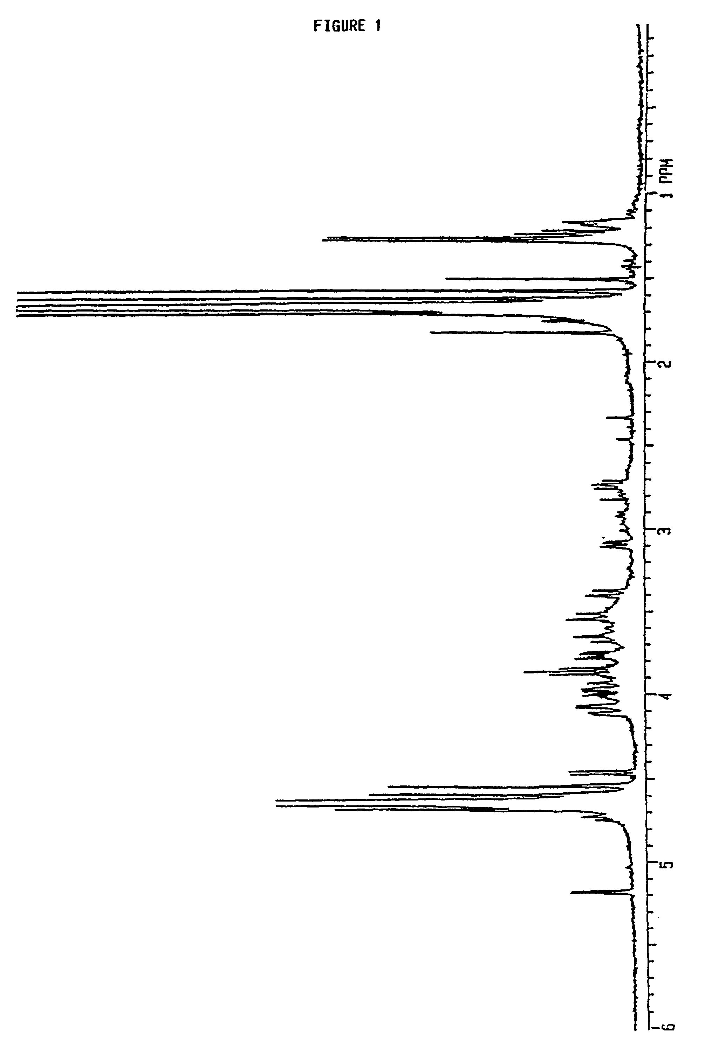

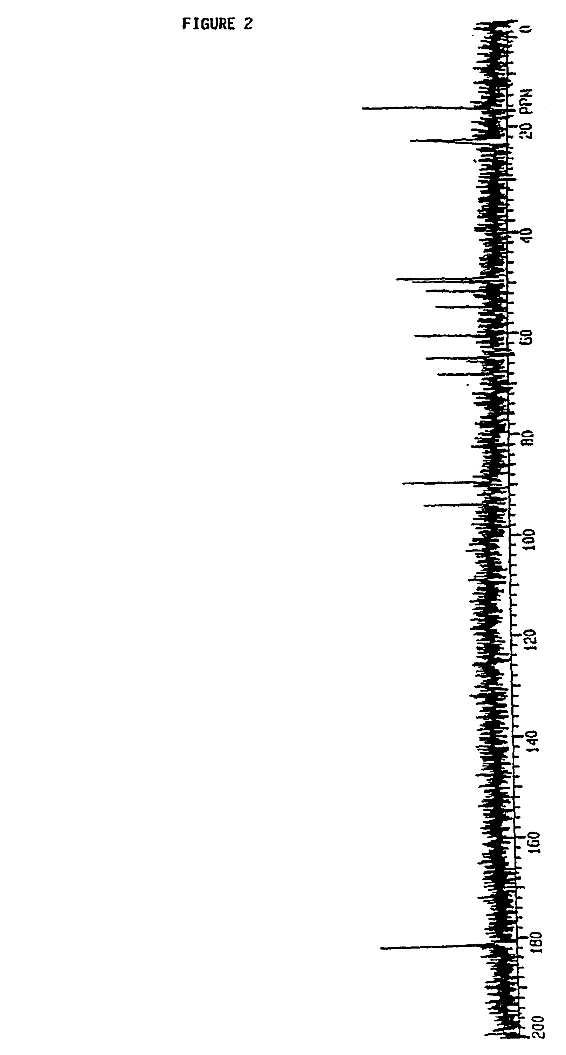

The active substance obtained from Example 3 is characterized. ¹H and ¹³C NMR spectroscopic data are collected on the purified substance using a 400 MHz Varian Gemini Spectrometer. The ¹H NMR (D₂O, 400 MHz) spectrum for the purified compound is shown in Figure 1 with ¹H shifts of about δ 5.17 (d,1H) 4.48 (d, 1H), 4.1 (m, 1H), 4.0 (dd, 1H), 3.92 (d, 1H), 3.85 (m, 2H), 3.80 (m, 1H), 3.65 (m, 1H), 3.50 (m, 1H), 3.36 (m, 1H), 3.1 (dd, 1H), 2.8 (m, 1H), 1.27 (d, 3H), and 1.19 (m, 3-4H). The ¹³C NMR (D₂O, 100 MHz) spectrum for the purified compound is shown in Figure 2 with ¹³C shifts of about δ 97.8, 92.5, 71.1, 68.1, 67.8, 63.6, 57.8, 54.7, 52.9, 52.7, 52.3, and 19.7.

The purified substance is further characterized with respect to its UV spectrum using a Hewlett-Packard Diode array detector and found to possess only UV end absorption. The substance is soluble methanol as well as water. Furthermore, the substance reacts positively with both ninhydrin and Dragendorff's reagents.

The purified subtance is estimated based on the NMR data to have a molecular weight in the range of about 250 to about 500.

### EXAMPLE 5: Determination of Pesticidal Substance's LC₅₀ and LC₉₀

The purified substance from the TSK SP-5PW HPLC purification step as described in EXAMPLE 3 is lyophilized once and redissolved in deionized water to a concentration of approximately 3 mg/ml. The substance is then submitted to bioassay against *Diabrotica undecimpunctata* to estimate the LC₅₀ and LC₉₀.

Artificial insect diet is prepared comprised of water, agar, sugar, casein, wheat germ, methyl paraben, sorbic acid, linseed oil, cellulose, salts, propionic acid, phosphoric acid, streptomycin, chlorotetracycline, and vitamins. The artificial diet allows samples consisting of rehydrated powders and liquids to be incorporated at a rate of 20% v/v. Test samples are prepared in microcentrifuge tubes to provide 8 to 16 serial dilutions. Liquid samples are tested at 200 µl/ml and then serially diluted in 0.1% TWEEN™ 20 to contain 100 µl/ml, 50 µl/ml, 25 µl/ml, 12.5 µl/ml, 6.25 µl/ml, 3.125 µl/ml, 1.563 µl/ml, etc. Each sample is mixed with molten diet to provide 1 ml. The molten mixture is vortexed and pipetted in 0.1 ml aliquots into 10 wells of a 96 well microtiter plate. Control samples containing 0.1% TWEEN™ 20 are dispensed into 16 wells. Once the diet solidifies, two neonate *Diabrotica undecimpunctata* larvae are added to each well, and the trays are covered with a perforated sheet of clear Mylar. The trays are incubated for 5 days at 28 ± 2°C and 65% relative humidity. The bioassays are replicated three times.

After 5 days, mortality is scored. The Mylar sheet is removed and each well is inspected using a dissecting microscope. Larvae that do not move when prodded with a dissecting needle are counted as dead. Percent mortality is calculated and the data analyzed via probit analysis. LC₅₀, LC₉₀, regression line slope, and coefficient of variation are estimated.

The substance is estimated to have an LC₅₀ of 20 µl/ml, a LC₉₀ of 172.2 µl/ml, a regression line slope of 1.5, and a coefficient of varnation of 18.8.

### DEPOSIT OF MICROORGANISM

The following strain of *Bacillus thuringiensis* has been deposited according to the Budapest Treaty in the Agricultural Research Service Patent Culture Collection Northern Regional Research Center (NRRL), 1815 University Street, Peoria, Illinois, 61604, USA.

| Strain | Accession Number | Deposit Date |
|---|---|---|
| EMCC-0080 | NRRL B-21093 | May 10, 1993 |

The strain has been deposited under conditions set out in Rule 28 EPC.

## Claims

1. A substance having pesticidal activity against a pest wherein the substance has
(a) activity against an insect pest of the order Coleoptera;
(b) 1H NMR (D₂O, 400 MHz) shifts of about δ 5.17 (d, 1H), 4.48 (d, 1H), 4.1 (m, 1H), 4.0 (dd, 1H), 3.92 (d, 1H), 3.85 (m, 2H), 3.80 (m, 1H), 3.65 (m, 1H), 3.50 (m, 1H), 3.36 (m, 1H), 3.1 (dd, 1H), 2.8 (m, 1H), 1.27 (d, 3H), and 1.19 (m, 3-4H);
(c) 12 carbons;
(d) ¹³C NMR (D₂O, 100MHz), shifts of about δ 97.8, 92.5, 71.1, 68.1, 67.8, 63.6, 57.8, 54.7, 52.9, 52.7, 52.3 and 19.7; and
(e) UV end absorption,
and is prepared from a culture of a *Bacillus thuringiensis* strain having the identifying characteristics of EMCC-0080, deposited with the NRRL, having an accession number of NRRL B-21093, or mutants thereof having substantially the same identifying characteristics of EMCC-0080.

2. The substance according to claim 1, wherein the substance has a molecular weight in the range of about 250 to about 500.

3. The substance according to claim 1, wherein the substance is water-soluble.

4. The substance according to claim 1, wherein the substance is obtained by
(a) culturing the *Bacillus thuringiensis* strain under suitable conditions;
(b) recovering a supernatant of the culture of the *Bacillus thuringiensis* strain of step (a); and
(c) isolating the substance from the supernatant of step (b).

5. The substance according to claim 1, wherein the insect pest is of the genus Diabrotica.

6. The substance according to claim 5, wherein the insect pest is *Diabrotica undecimpunctata*.

7. A method for obtaining a substantially pure substance of claim 1 comprising
(a) culturing a *Bacillus thuringiensis* strain having the identifying characteristics of EMCC-0080, deposited with the NRRL, having an accession number of NRRL B-21093, or mutants thereof having substantially the same identifying characteristics of EMCC-0080 under suitable conditions;
(b) recovering a supernatant of the culture of the *Bacillus* strain of step (b); and
(c) isolating the substance from the supernatant of step (b) to obtain the substantially pure substance.

8. The method according to claim 7, wherein the substance is isolated from the supematant by column chromatography.

9. A pesticidal composition comprising (a) the substance of claim 1 and (b) a pesticidal carrier, wherein the substance is present in an amount effective for controlling a pest.

10. The pesticidal composition according to claim 9, wherein the substance is present in the composition in an amount in the range of about 0.001% by weight to about 60% by weight.

11. A method for controlling a pest comprising exposing the pest to a pesticidally-effective amount of the pesticidal composition of claim 9.

12. A method for obtaining a mutant of a *Bacillus thuringiensis* strain EMCC-0080 wherein the amount of the substance of claim 1 produced by the mutant is greater than the amount of the substance produced by a corresponding parental strain, comprising
(a) treating the *Bacillus thuringiensis* strain EMCC-0080 with a mutagen;
(b) growing the mutated *Bacillus* strain of step (a) in a medium suitable for selecting the mutant strain; and
(c) selecting the mutant strain of step (b) on the basis of increased production of the substance.

13. A mutant of the *Bacillus thuringiensis* strain EMCC-0080 obtained according to the method of claim 12.

## Patentansprüche

1. Eine Substanz, die eine pestizide Wirksamkeit gegen einen Schädling besitzt, worin die Substanz
(a) Wirksamkeit gegen einen Schädling der Ordnung Coleoptera besitzt;
(b) 1H NMR (D₂O, 400 Mhz) Verschiebungen von ungefähr δ 5.17 (d, 1H), 4,48 (d, 1H), 4,1 (m, 1H), 4,0 (dd, 1H), 3,92 (d, 1H), 3,85 (m, 2H), 3,80 (m, 1H), 3,65 (m, 1H), 3,50 (m, 1H), 3,36 (m, 1H), 3,1 (dd, 1H), 2,8 (m, 1H), 1,27 (d, 3H), und 1,19 (m, 3-4H) besitzt;
(c) 12 Kohlenstoffe besitzt;
(d) ¹³C NMR (D₂O, 100 Mhz) Verschiebungen von ungefähr δ 97,8, 92,5, 71,1, 68,1, 67,8, 63,6, 57,8, 54,7, 52,9, 52,7, 52,3 und 19,7 besitzt; und
(e) UV Endabsorption besitzt,
und hergestellt wird aus einer Kultur eines *Bacillns thuringiensis* Stamms mit den Identifikationscharakteristika von EMCC-0080, hinterlegt mit den NRRL, mit einer Zugriffsnummer von NRRL B-21093, oder Mutanten davon, die im wesentlichen dieselben Charakteristika von EMCC-0080 besitzen.

2. Die Substanz gemäß Anspruch 1, worin die Substanz ein Molekulargewicht im Bereich von ungefähr 250 bis ungefähr 500 besitzt.

3. Die Substanz gemäß Anspruch 1, worin die Substanz wasserlöslich ist.

4. Die Substanz gemäß Anspruch 1, worin die Substanz erhalten wird durch
(a) Kultivieren des *Bacillus thuringiensis* Stamms unter geeigneten Bedingungen;
(b) Gewinnen eines Überstandes der Kultur des *Bacillus thuringiensis* Stamms aus Schritt (a); und
(c) Isolieren der Substanz aus dem Überstand aus Schritt (b).

5. Die Substanz gemäß Anspruch 1, worin der Schädling der Gattung Diabrotica angehört.

6. Die Substanz gemäß Anspruch 5, worin der Schädling *Diabrotica undecimpunctata* ist.

7. Ein Verfahren zum Erhalten einer im wesentlichen reinen Substanz gemäß Anspruch 1, das folgendes umfaßt:
(a) Kultivieren eines *Bacillus thuringiensis* Stamms, der die Identifikationscharakteristika von EMCC-0800 besitzt, hinterlegt mit den NRRL, mit einer Zugriffsnummer von NRRL B-21093, oder Mutanten davon, die im wesentlichen dieselben Identifikationscharakteristika von EMCC-0800 besitzen, unter geeigneten Bedingungen;
(b) Gewinnen eines Überstandes der Kultur des *Bacillus*-Stamms von Schritt (b); und
(c) Isolieren der Substanz aus dem Überstand von Schritt (b), um die im wesentlichen reine Substanz zu erhalten.

8. Das Verfahren gemäß Anspruch 7, worin die Substanz aus dem Überstand durch Säulenchromatographie isoliert wird.

9. Eine pestizide Zusammensetzung, die (a) die Substanz von Anspruch 1 und (b) einen pestiziden Träger umfaßt, worin die Substanz in einer Menge anwesend ist, die wirksam ist zur Bekämpfung eines Schädlings.

10. Die pestizide Zusammensetzung gemäß Anspruch 9, worin die Substanz in der Zusammensetzung in einer Menge im Bereich von ungefähr 0,001 Gew% bis ungefähr 60 Gew% anwesend ist.

11. Ein Verfahren zur Bekämpfung eines Schädlings, das das Aussetzen des Schädlings einer pestizid-wirksamen Menge der pestiziden Zusammensetzung von Anspruch 9 umfaßt.

12. Ein Verfahren zum Erhalten eines Mutanten eines *Bacillus thuringiensis* Stamms EMCC-0800, worin die Menge der Substanz von Anspruch 1, die durch den Mutanten produziert wird, größer ist als die Menge der Substanz, die durch einen entsprechenden elterlichen Stamm produziert wird, das folgendes umfaßt:
(a) Behandeln des *Bacillus thuringiensis* Stamms EMCC-0800 mit einem Mutagen;
(b) Züchten des mutierten *Bacillus* Stamms aus Schritt (a) in einem Medium, das geeignet ist, um den Mutantenstamm zu selektieren; und
(c) Selektieren des Mutantenstamms aus Schritt (b) auf der Basis von erhöhter Produktion der Substanz.

13. Ein Mutant des *Bacillus thuringiensis* Stamms EMCC-0800, der gemäß dem Verfahren von Anspruch 12 erhalten wurde.

## Revendications

1. Substance ayant une activité pesticide contre un insecte dans laquelle la substance présente
(a) une activité contre un insecte de la classe Coleoptera ;
(b) la RMN-¹H (D₂O, 400 MHz) se déplace d'environ δ 5,17 (d, 1H), 4,48 (d, 1H), 4,4 (m, 1H), 4,0 (dd, 1H), 3,92 (d, 1H), 3,85 (m, 2H), 3,80 (m, 1H), 3,65 (m, 1H), 3,50 (m, 1H), 3,36 (m, 1H), 3,1 (dd, 1H), 2,8 (m, 1H), 1,27 (d, 3H) et 1,19. (m, 3-4H) ;
(c) 12 atomes de carbone ;
(d) la RMN-¹³C (D₂O, 100 MHz) se déplace d'environ δ 97,8, 92,5, 71,1, 68,1, 67,8, 63,6, 57,8, 54,7, 52,9, 52,7, 52,3 et 19,7 ; et
(e) une absorption finale UV
et est préparée à partir d'une culture d'une souche de *Bacillus thuringiensis* ayant les caractéristiques d'identification de EMCC-0080, déposées avec la NRRL, ayant un numéro d'accession de NRRL B-21093, ou de mutants de celle-ci ayant pratiquement les mêmes caractéristiques d'identification de EMCC-0080.

2. Substance selon la revendication 1, dans laquelle la substance présente un poids moléculaire dans l'intervalle d'environ 250 à environ 500.

3. Substance selon la revendication 1, dans laquelle la substance est soluble dans l'eau.

4. Substance selon la revendication 1, dans laquelle la substance est obtenue par
(a) la mise en culture de la souche de *Bacillus thunngiensis* dans des conditions appropriées ;
(b) la récupération d'une matière surnageante de la culture de la souche de *Bacillus thuringiensis* de l'étape (a) ; et
(c) l'isolement de la substance à partir de la matière surnageante de l'étape (b).

5. Substance selon la revendication 1, dans laquelle l'insecte est du genre Diabrotica.

6. Substance selon la revendication 5, dans laquelle l'insecte est *Diabrotica undecimpunctata*.

7. Procédé pour l'obtention d'une substance pratiquement pure selon la revendication 1 comprenant
(a) la mise en culture d'une souche de *Bacillus thuringiensis* ayant les caractéristiques d'identification de EMCC-0080, déposées avec la NRRL, ayant un numéro d'accession de NRRL B-21093, ou de mutants de celle-ci ayant pratiquement les mêmes caractéristiques d'identification de EMCC-0080 dans des conditions appropriées ;
(b) la récupération d'une matière surnageante de la culture de la souche de *Bacillus* de l'étape (b) ; et
(c) l'isolement de la substance à partir de la matière surnageante de l'étape (b) pour obtenir la substance pratiquement pure.

8. Procédé selon la revendication 7, dans lequel la substance est isolée à partir de la matière surnageante par chromatographie sur colonne.

9. Composition pesticide comprenant (a) la substance selon la revendication (1) et (b) un véhicule pesticide, dans laquelle la substance est présente dans une quantité efficace pour contrôler un insecte.

10. Composition pesticide selon la revendication 9, dans laquelle la substance est présente dans la composition dans une quantité dans l'intervalle d'environ 0,001 % en poids à environ 60 % en poids.

11. Procédé pour contrôler un insecte comprenant l'exposition de l'insecte à une quantité efficace en pesticide de la composition pesticide selon la revendication 9.

12. Procédé pour l'obtention d'un mutant d'une souche de *Bacillus thuringiensis* EMCC-0080 dans lequel la quantité de la substance selon la revendication 1 produite par le mutant est supérieure à la quantité de la substance produite par une souche parentale correspondante comprenant
(a) le traitement de la souche de *Bacillus thuringiensis* EMCC 0080 avec un mutagène ;
(b) la croissance de la souche de *Bacillus* mutée de l'étape (a) dans un milieu approprié pour choisir la souche de mutant ; et
(c) le choix de la souche de mutant de l'étape (b) sur la base d'une production plus élevée de la substance.

13. Mutant de la souche de *Bacillus thuringiensis* EMCC-0080 obtenu selon le procédé de la revendication 12.
